# EUROPEAN PATENT APPLICATION

(11) **EP 1 637 078 A1**
(43) Date of publication of application: **22.03.2006**
(21) Application number: 04746537.2
(22) Date of filing: 22.06.2004
(51) Int. Cl.: A61B 5/15, A61B 5/145, G01N 1/10

(54) **BODY FLUID SAMPLING IMPLEMENT AND BODY FLUID SAMPING METHOD**

(30) Priority: 23.06.2003 JP 2003178749
(71) Applicant: TERUMO KABUSHIKI KAISHA, Tokyo 151-0072 (JP)
(72) Inventor: TAKINAMI, Masao, Terumo Kabushiki Kaisha, Nakakoma-gun, Yamanashi 409-3853 (JP); TAKEMOTO, Masafumi, Terumo Kabushiki Kaisha, Nakakoma-gun, Yamanashi 409-3853 (JP)
(74) Representative: Leson, Thomas Johannes Alois
(86) International application number: PCT/JP2004/009068
(87) International publication number: WO 2004/112613

(57) **Abstract**

A component measuring apparatus (body fluid sampling implement in this invention) used by installing a piercing needle therein and allowing blood (body fluid) to be discharged by piercing a blood sampled portion (body fluid sampled portion) with the piercing needle, comprising a body storing the piercing needle and having an opening allowing the piercing needle to pass therethrough and a contact part fixedly installed on the body so as to surround the outer periphery of the opening and pressed by the blood sampled portion when the blood sampled portion is pierced by the piercing needle. The contact part has a function to specify a shape near the blood sampled portion when the blood sampled portion is pressed against the contact portion. In addition, the contact surface (inner surface) of the contact part (3) is tilted so as to near the center axis of the opening toward the opening of the body and formed in the shape of a truncated cone in side view.

## Description

### Technical Field

The present invention relates to a body fluid sampling implement and a body fluid sampling method.

### Background Art

In recent years, attendant on the increase in the number of diabetics, blood sugar self-measurement in which daily variations in blood sugar level is monitored by the patient himself has been recommended.

This measurement of blood sugar level is conducted by use of a blood sugar measuring instrument in which a test paper showing coloration according to the amount of blood sugar in blood is mounted, blood is supplied to and developed on the test paper to cause the coloration, and the degree of the coloration is optically measured (colorimetry) to thereby quantitatively determine the blood sugar level.

As a method for the patient to sample his own blood before the measurement, there is adopted a method in which the skin of a fingertip is pierced by a piercing implement having a piercing needle or a small blade, and then the surroundings of the pierced portion are depressed by a finger or the like to squeeze the blood out.

However, a fingertip is suited to blood sampling because blood capillaries are concentratedly present there, but, on the other hand, nerves are also concentratedly present there, so that the blood sampling is attended by an ache. Thus, the blood sampling method exerts great pain and burden on the patient, and, in addition, the piercing arise a sensation of fear; accordingly, many of the patients may be unable to continue the blood sugar self-measurement.

As an apparatus capable of solving such a problem, there has been developed a blood sugar measuring instrument described in Japanese Patent Laid-Open No. 2001-309905, namely, a blood sugar measuring instrument in which a piercing device and a measuring device are integrated with each other and which has suction means for squeezing blood out.

In using the blood sugar measuring instrument described in Japanese Patent Laid-Open No. 2001-309905, first, a chip provided with a test paper is mounted to an instrument body, and a fingertip is pressed against the tip end of the chip so as to seal the tip end opening in a gas-tight manner.

Next, the fingertip is pierced with a piercing needle which projects from the tip end opening, and, in this condition, the suction means is operated (to produce a reduced pressure condition), thereby sucking out blood from the pierced portion and sampling the blood. Then, the blood sugar level in the blood thus sampled is measured on the measuring device.

However, such a blood sugar measuring instrument has the problem that, while the instrument is used by pressing the fingertip to the tip end of the chip, it is difficult to make constant the force with which the finger tip is pressed against the tip of the chip each time of measurement of blood sugar. This causes the problem that, in the conventional blood sugar measuring instruments, there arises a dispersion in the depth of piercing of the fingertip with the piercing needle, with the result that accurate measurement of blood sugar cannot be achieved in the case where the blood is not sampled in an amount necessary for the blood sugar measurement.

### Disclosure of Invention

It is an object of the present invention to provide a body fluid sampling implement and a body fluid sampling method by which a body fluid can be sampled more securely.

In order to attain the above object, according to the present invention, there is provided a body fluid sampling implement used by installing a piercing needle therein and allowing a body fluid to be discharged by piercing a body fluid sampled portion with the piercing needle, including:
a body storing the piercing needle and having an opening allowing the piercing needle to pass therethrough; and
a contact part fixedly installed on the body so as to surround the outer periphery of the opening and pressed by the body fluid sampled portion when the body fluid sampled portion is pierced by the piercing needle,
wherein the inner surface of the contact part has a portion tilted so as to near the center axis of the opening as the opening is approached.

This ensures that when the body fluid sampled portion is pressed against the contact part, the surroundings of the body fluid sampled portion are deformed along the shape of the inner surface of the contact part, and is determined in shape so that a portion thereof is projected into the body through the opening. Therefore, the depth of piercing of the body fluid sampled portion by the piercing needle can be made to be substantially constant. As a result, a dispersion in the depth of piercing by the piercing needle is suppressed, and it becomes possible to sample a body fluid more accurately.

In the body fluid sampling implement according to the present invention, the inner surface of the contact part is preferably in the shape of a truncated cone.

In the body fluid sampling implement according to the present invention, it is preferable that the piercing needle is mounted to the body fluid sampling implement as a chip stored in a chip body having an opening allowing the piercing needle to pass therethrough, and the body fluid sampled portion abuts on an edge portion of the opening of the chip body when the body fluid sampled portion is pressed against the contact part in the condition where the chip is mounted to the body fluid sampling implement.

In the body fluid sampling implement according to the present invention, an opening area of the opening of the chip body is preferably smaller than an opening area of the opening of the body.

In the body fluid sampling implement according to the present invention, it is preferable that the edge portion of the opening of the chip body is located substantially on an extension plane of the tilted portion of the inner surface of the contact part in the condition before the body fluid sampled portion is pierced by the piercing needle.

In the body fluid sampling implement according to the present invention, the body fluid sampling implement is preferably used in the condition where said opening of said body is directed vertically upward.

According to the present invention, there is provided a body fluid sampling method for sampling a body fluid by piercing a body fluid sampled portion with a piercing needle by use of a body fluid sampling implement including:
a body storing the piercing needle and having an opening allowing the piercing needle to pass therethrough; and
a contact part fixedly installed on the body so as to surround the outer periphery of the opening and pressed by the body fluid sampled portion when the body fluid sampled portion is pierced by the piercing needle,
the inner surface of the contact part having a portion tilted so as to near the center axis of the opening as the opening is approached,
wherein the body fluid is discharged by piercing the body fluid sampled portion with the piercing needle in the condition where the body fluid sampled portion is pressed against the contact part.

According to the present invention, there is provided a body fluid sampling method carried out by use of a body fluid sampling implement including:
a body storing the piercing needle and having an opening allowing the piercing needle to pass therethrough; and
a contact part fixedly installed on the body so as to surround the outer periphery of the opening and pressed by the body fluid sampled portion when the body fluid sampled portion is pierced by the piercing needle,
the inner surface of the contact part having a portion tilted so as to near the center axis of the opening as the opening is approached,
wherein the method includes the steps of:
pressing the body fluid sampled portion into close contact with the inner surface of the contact part;
operating the piercing needle so as to pierce with the piercing needle that portion of the body fluid sampled portion which is projected into the body through the opening; and
sampling the body fluid discharged from the pierced portion of the body fluid sampled portion.

In the body fluid sampling method according to the present invention, the inner surface of the contact part is preferably in the shape of a truncated cone.

In the body fluid sampling method according to the present invention, it is preferable that the piercing needle is mounted to the body fluid sampling implement as a chip stored in a chip body having an opening allowing the piercing needle to pass therethrough, and
the body fluid sampled portion abuts on an edge portion of the opening of the chip body when the body fluid sampled portion is pressed against the contact part in the condition where the chip is mounted to the body fluid sampling implement.

In the body fluid sampling method according to the present invention, the opening area of the opening of the chip body is smaller than the opening area of the opening of the body.

In the body fluid sampling method according to the present invention, it is preferable that the edge portion of the opening of the chip body is located substantially on an extension plane of the tilted portion of the inner surface of the contact part in the condition before the body fluid sampled portion is pierced by the piercing needle.

In the body fluid sampling method according to the present invention, the body fluid sampling implement is preferably used in the condition where the opening of the body is directed vertically upward.

### Brief Description of Drawings

Fig. 1 is a perspective view showing an embodiment (the condition where a cover is opened) in the case where the body fluid sampling implement according to the present invention is applied to a component measuring apparatus.
Fig. 2 is a side view of the component measuring apparatus (the condition where the cover is opened) shown in Fig. 1.
Fig. 3 is a vertical sectional view showing the configuration of an essential part of the component measuring apparatus according to the present invention.
Fig. 4 is a vertical sectional view showing the configuration of an essential part of the component measuring apparatus according to the present invention.
Fig. 5 is a block diagram showing a circuit configuration of the component measuring apparatus shown in Fig. 1.
Fig. 6 is a flowchart showing a control action (partly inclusive of the operator's operations and the like) of control means in the component measuring apparatus shown in Fig. 1.

### Best Mode for Carrying out the Invention

Now, the body fluid sampling implement and the body fluid sampling method according to the present invention will be described in detail below, based on preferred embodiments shown in the accompanying drawings.

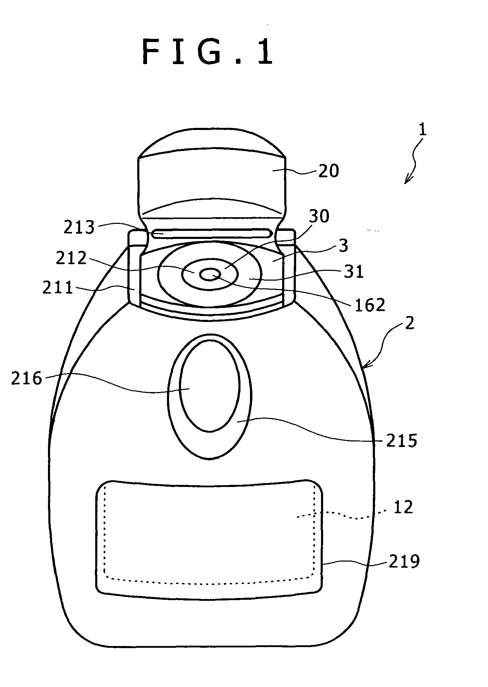

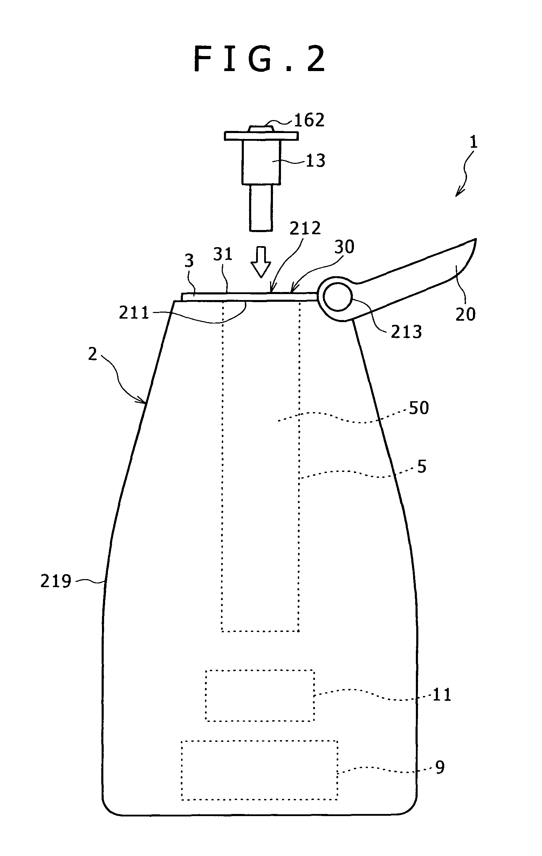

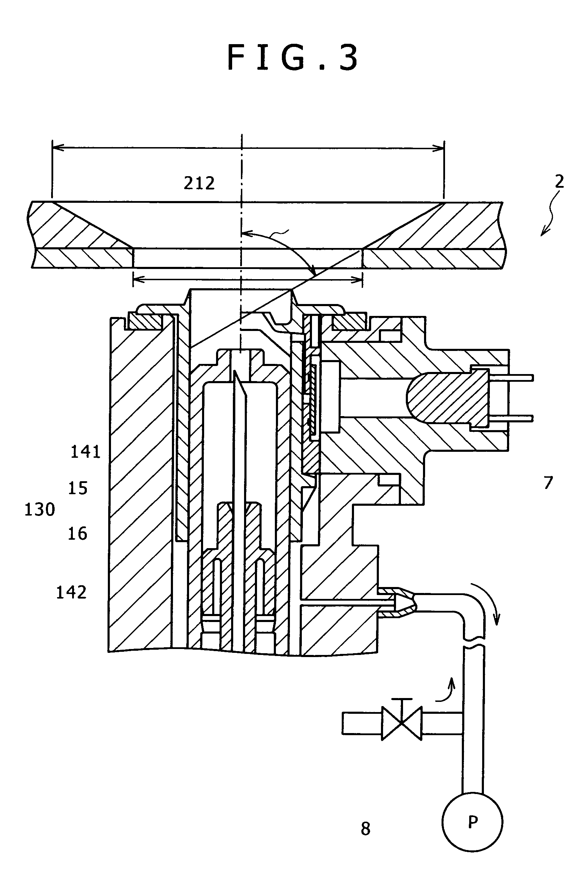

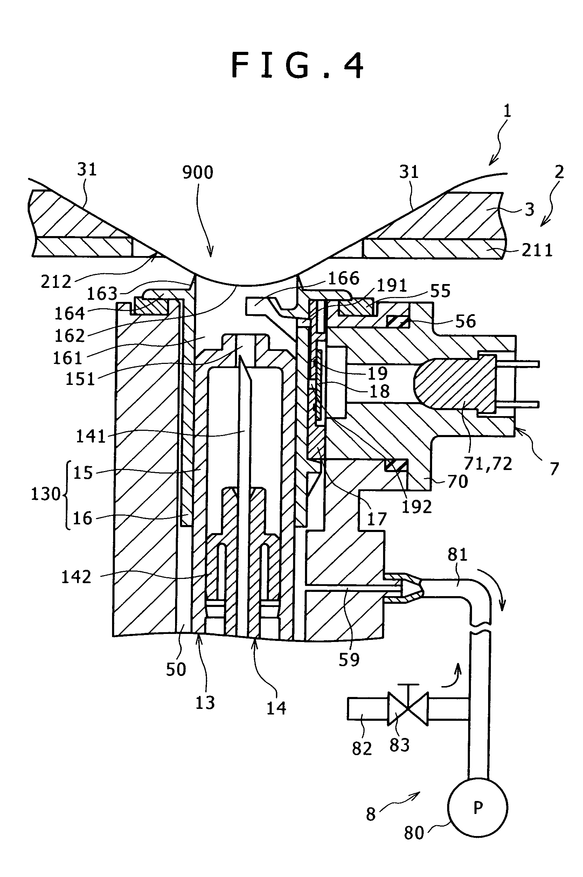

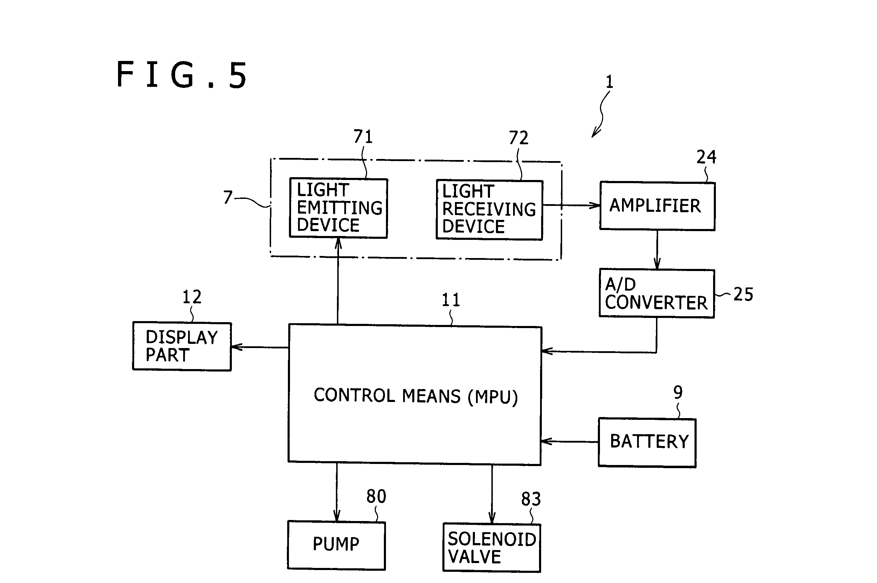

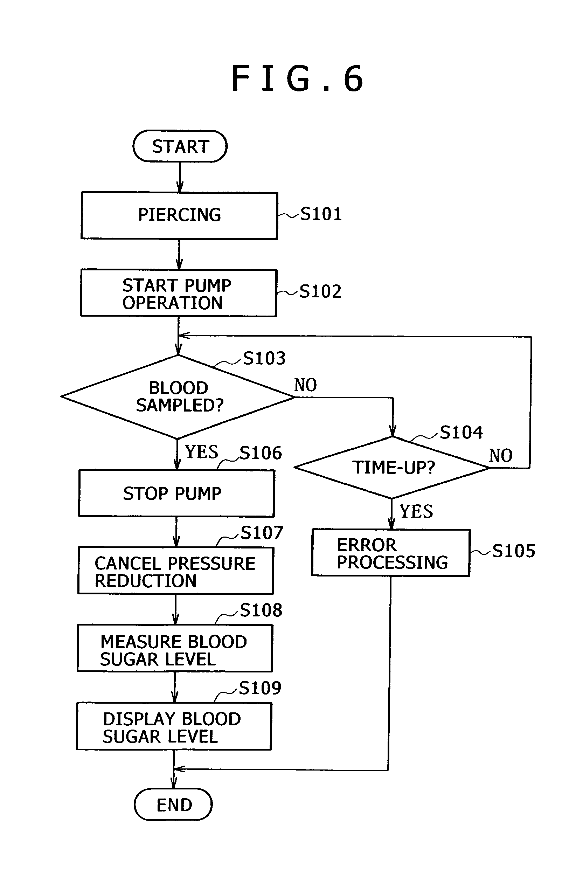

Fig. 1 is a perspective view showing an embodiment (the condition where a cover is opened) in the case where the body fluid sampling implement according to the present invention is applied to a component measuring apparatus; Fig. 2 is a side view of the component measuring apparatus shown in Fig. 1 (the condition where the cover is opened); Figs. 3 and 4 are each a vertical side view showing the configuration of an essential part of the component measuring apparatus according to the present invention; Fig. 5 is a block diagram showing the circuit configuration of the component measuring apparatus shown in Fig. 1; and Fig. 6 is a flowchart showing a control action (partly inclusive of the operator's operations and the like) of control means in the component measuring apparatus shown in Fig. 1. Incidentally, in Figs. 1 to 4, description will be made by deeming the upper side as "distal" or "upper", and the lower side as "proximal", "lower" or "bottom".

A component measuring apparatus (blood component measuring apparatus) 1 shown in each of the drawings is an apparatus for sampling a body fluid (hereinafter, in this embodiment, blood is described as a representative thereof) and measuring the quantity of a predetermined component of the body fluid.

The portion used for blood sampling in use of the component measuring apparatus 1 may be, for example, a palm, the back of a hand, a side portion of a hand, a finger (fingertip), an arm, abdomen, a thigh, an earlobe, or the like; in the following embodiments, the mode in which a side portion of a hand is pierced will be described as a representative.

As shown in Figs. 1 and 2, the component measuring apparatus 1 includes a body 2, a cover 20 disposed so as to be turnable (openable and closable) relative to the body, a housing 5 disposed inside the body 2, measuring means 7 for detecting the sampling of blood and measuring a predetermined component in the blood thus sampled, pressure reducing means 8 for putting the inside of the housing into a reduced pressure state, control means 11 provided on a circuit substrate which is not shown, and a display part 12. The component measuring apparatus 1 is used in the condition where a chip 13 is installed in a chip installing part (chip installing space) 50 formed in the housing. Now, each of component elements will be described below.

The body 2 is box-like in shape, and stores therein the housing 5, the measuring means 7, the pressure reducing means 8, a battery (power supply part) 9, the control means 11, the display part 12, and the like.

A wall part 211 on the distal side (upper side) of the body 2 is provided with an opening 212 which has a roughly circular shape in cross-section. The component measuring apparatus 1 in this embodiment is of a vertical installation type to be used in the condition where the opening 212 is directed vertically upward, and the chip 13 is installed (held) into the chip installing part 50 formed in the housing 5 via the opening 212.

In addition, a contact part 3 against which a blood sampled portion (body fluid sampled portion) 900 of the side portion of a hand is to be pressed is fixed (or attached) to the surface on the distal side of the wall part 211 so as to surround the outer periphery of the opening 212 by such a method as fitting, screw engagement, adhesion with an adhesive, et al. Incidentally, the contact part 3 may be formed as one body with the body 2.

In the condition where the chip 13 is installed in the chip installing part 50 (hereinafter referred to as "the chip installed condition"), the component measuring apparatus 1 is operated while keeping the blood sampled portion 900 in abutment on the contact part 3. By this, the surface (skin) of the blood sampled portion 900 is pierced, and the quantity of a predetermined component (hereinafter, in this embodiment, glucose will be described as a representative) in the blood sampled is measured. Incidentally, while the present invention is characterized in that the contact part 3 is provided, this characteristic will be described in detail later.

In addition, the cover 20 is turnably supported on a distal end portion (upper portion) of the body 2 through a shaft (rotating shaft) 213.

With the cover 20 closed, the distal end of the body and the contact part 3 are covered by the cover 20. On the other hand, with the cover 20 opened as shown in Figs. 1 and 2, the distal end of the body 2 and the contact part 3 are exposed to the outside, so that attachment and detachment of the chip 13, measurement, and the like can be carried out.

The surface on the front side of the body 2 is provided with a display window (opening) 219 penetrating through the body 2 between the inside and the outside, and the display window 219 is closed with a plate-like member composed of a transparent material. The display part 12 is installed on the inside of the display window 219. Therefore, various kinds of information displayed on the display part 12 can be confirmed through the display window 219.

The display part 12 is composed, for example, of a liquid crystal display device (LCD) or the like. For example, ON/OFF of a power supply, the power supply voltage (residual amount of a battery), measurements, measurement date and time, error indications, operation guidance, and the like can be displayed on the display part 12.

In addition, the surface on the front side of the body 2 is provided with a recessed portion 215 on the distal side (upper side) thereof, and an operating button 216 is installed in the recessed portion 215. In the component measuring apparatus 1 according to this embodiment, a configuration is adopted in which, with the operating button 216 depressed, piercing means and a pump 80 as the pressure reducing means 8 which will be described later are operated sequentially or roughly simultaneously. Incidentally, a configuration may be adopted in which, with the operating button 216 depressed, the power supply for the component measuring apparatus 1 is turned ON.

The control means 11 is composed, for example, of a microcomputer, and controls various operations of the component measuring apparatus 1, such as discrimination of whether blood has been sampled or not. Besides, the control means 1 incorporates an arithmetic unit for calculating the glucose level (blood sugar level) based on a signal from the measuring means 7.

The housing has, in its inside, the chip installing part 50 for installing the chip 13. An annular seal ring (sealing member) 55 is fitted to the distal end of the housing 5, i.e., to an outer peripheral portion of the distal end opening of the chip installing part 50. This ensures that, with the chip 13 mounted in the chip installing part 50, a flange 164 of the chip 13 is brought into close contact with the seal ring 55, whereby gas-tightness of the chip installing part 50 is maintained.

The seal ring 55 is composed of an elastic body (elastic material). Examples of the elastic body include various rubber materials such as natural rubber, isoprene rubber, butadiene rubber, styrene-butadiene rubber, nitrile rubber, chloroprene rubber, butyl rubber, acrylic rubber, ethylene-propylene rubber, hydrin rubber, urethane rubber, silicone rubber, fluoro-rubber, etc., and various thermoplastic elastomers based on styrene, polyolefin, polyvinyl chloride, polyurethane, polyester, polyamide, polybutadiene, fluoro-rubber or the like.

The measuring means 7 is installed at a side portion of the housing 5. The measuring means 7 is for optically detecting the supply (sampling) of blood onto a test paper 18 possessed by the chip 13, and for optically measuring the quantity of glucose in the blood developed on the test paper 18. The measuring means 7 is composed of an optical block, and is installed in a position facing the test paper 18 in the chip installed condition (the lateral vicinity of the test paper 18).

Thus, the measuring means 7 has both the function to detect the sampling of blood and the function to measure the quantity of glucose (predetermined component) in the blood developed on the test paper 18. Therefore, it is possible to reduce the number of component parts, to simplify the configuration, and to reduce the assembling steps of the apparatus, as compared with the case where the individual means for these functions are provided separately.

The measuring means 7 has a block body 70, and a light emitting device (light emitting diode) 71 and a light receiving device (photo-diode) 72 which are fixed to the block body 70.

A seal ring 56 similar to the seal ring 55 is disposed between the block body 70 and the housing 5, for maintaining the gas-tightness of the chip installing part 50.

The light emitting device 71 is electrically connected to the control means 11, and the light receiving device 72 is electrically connected to the control means 11 through an amplifier 24 and an A/D converter 25 (see Fig. 5).

The light emitting device 71 is operated by a signal from the control means 11, to emit light. The light is preferably pulsed light which is intermittently emitted at a predetermined time interval.

With the light emitting device 71 turned ON in the chip installed condition, the test paper 18 is irradiated with the light emitted from the light emitting device 71, and the reflected light is received by the light receiving device 72, to undergo photo-electric conversion. The light receiving device 72 outputs an analog signal according to the quantity of light received. The signal is amplified in a desired manner by the amplifier 24, the amplified signal is then converted into a digital signal by the A/D converter 25, and the digital signal is inputted to the control means 11.

In the control means 11, whether blood has been sampled or not, i.e., whether the blood has been developed on the test paper 18 of the chip 13 or not is discriminated, based on the signal inputted.

In addition, the control means 11 performs a predetermined arithmetic processing, based on the signal inputted, and performs a correcting calculation or the like as required, to determine the quantity of glucose in the blood (blood sugar level). The blood sugar level thus obtained is displayed on the display part 12.

Now, the configuration of the chip 13 to be installed in the chip installing part 50 will be described. As shown in Fig. 3, the chip 13 is composed of a piercing needle 14, an inner tube 15 for slidably storing the piercing needle 14, an outer tube 16 disposed at an outer peripheral portion of the inner tube 15, a test paper fixing part 17 disposed at an outer peripheral portion of the outer tube 16, and the test paper 18 fixed to the test paper fixing part 17. Of these component parts, the inner tube 15 and the outer tube 16 constitute a chip body 130.

The piercing needle 14 is composed of a needle body 141, and a hub 142 attached to the proximal side of the needle body 141, and is stored in a lumen portion of the inner tube 15.

The needle body 141 is composed of a hollow member or solid member formed of a metallic material, for example, stainless steel, aluminum, an aluminum alloy, titanium, or a titanium alloy, and is provided at its distal end with a sharp cutting edge (needle tip). The cutting edge is used to pierce the surface (skin) of the blood sampled portion 900 of a side portion of a hand.

In addition, the hub 142 is composed of a member which is roughly hollow cylindrical or circular columnar in shape.

A hole 151 having a circular shape in cross-section is formed in a roughly central portion of the distal end of the inner tube 15. The needle body 141 passes through the hole 151 at the time of piercing the blood sampled portion 900. In addition, the diameter of the hole 151 is set to be smaller than the outside diameter of the distal end of the hub 142. Therefore, when the piercing needle 14 is moved in the inner tube 15 in the distal direction, the hub 142 cannot pass through the hole 151, and abuts on the periphery of the hole 151, whereby the piercing needle 14 is prevented from moving further in the distal direction. Therefore, the length by which the needle body 141 project from the distal end of the chip 13 at the time of piercing the blood sampled portion 900 is kept constant.

The outer tube 16 is attached to an outer peripheral portion of the inner tube 15. The outer tube 16 is composed of a roughly hollow cylindrical member, and has a lumen portion 161 therein.

The outer tube 16 is provided at its distal end with a contact part 163 projected in the shape of a ring. The contact part 163 is provided on its inside with a distal opening (opening) 162 where the lumen portion of the outer tube 16 (the lumen portion of the chip body 130) opens.

As will be described later, in this embodiment, at the time of piercing the blood sampled portion 900, the blood sampled portion 900 is pressed against the contact part 3, whereon the blood sampled portion 900 is pressed also against the contact part 163 so that the distal opening 162 is closed by the blood sampled portion 900.

An outer peripheral edge of the contact part 163 (an edge portion of the distal opening 162) has a shape suitable for displaying an effect of alleviating the ache at the time of piercing, by stimulating the surroundings of the pierced portion when pressed against the blood sampled portion 900. In addition, the shape is so set that, when the chip installing part 50 is brought into a reduced pressure state by the pressure reducing means 8 (described later), inflow of air through the gap between the distal end of the contact part 163 and the surface of the blood sampled portion 900 is restrained as securely as possible.

Besides, the outer tube 16 is provided with an annular flange 164 projecting outward, at an outer peripheral portion near the proximal end of the contact part 163. In the chip installed condition, the proximal surface of the flange 164 abuts on the distal end of the housing 5, whereby the chip 13 is positioned relative to the housing 5 (positioned in the vertical direction in Figs. 3 and 4).

The outer tube 16 is provided at its outer peripheral portion with the test paper fixing portion (test paper fixing member) 17, and the test paper 18 is fixed to the test paper fixing part 17.

In addition, the outer tube 16 is provided at its inner peripheral surface with a blood introduction guide 166 projecting toward a central portion of the lumen portion 161. The blood introduction guide 166 has the function to receive the blood (specimen) flowing out of a hand (blood sampled portion 900) into the lumen portion 161 after the piercing.

Besides, the chip 13 is provided with a blood passage 19 formed to penetrate through the outer tube 16 and the test paper fixing part 17. The blood passage 19 is a conduit for introducing the blood obtained by the piercing to the test paper 18, and has a passage opening 191 opening into the lumen portion 161 and a passage opening 192 opening to the outside of the chip 13. The passage opening 192 is located at a central portion of the test paper 18.

In addition, the passage opening 191 is formed near a proximal portion of the blood introduction guide 166. Therefore, the blood received by the blood introduction guide 166 is efficiently guided through the passage opening 191 into the blood passage 19. The blood reaches the passage opening 192 by capillary action, is supplied to a central portion of the test paper 18 disposed so as to cover the passage opening 192, and is developed radially on the test paper 18.

The test paper 18 has a reagent supported on a support capable of absorbing and developing blood.

Examples of the support include sheet-like porous bodies such as nonwoven fabric, woven fabric, and oriented sheet. The porous body is preferably hydrophilic.

The reagent supported on the support is appropriately determined according to the component to be measured contained in the blood (specimen). In the case for measurement of blood sugar level, examples of the reagent include glucose oxidase (GOD), peroxidase (POD), and color formers (color development reagents) such as 4-aminoantipyrine, and N-ethyl-N-(2-hydoroxy-3-sulopropyl)-m-toluidine. Other examples, according to the component to be measured, include reagents capable of reacting with the blood component, such as ascorbate oxidase, alcohol oxidase, and cholesterol oxidase and the same color formers (color development reagents) as above. Further, a buffer such as phosphoric acid buffer may be contained in the reagent. Incidentally, the kind and component of the reagent are naturally not limited to the just-mentioned ones.

In addition, the pressure reducing means (suction means) 8 capable of bringing the inside of the housing 5 (chip installing part 50) into a reduced pressure state is disposed in the body 2. The pressure reducing means 8 is composed of a pump 80, a pipe 81, a branch pipe 82 branched from an intermediate portion of the pipe 81 and opened at its distal end, and a solenoid valve 83 provided in an intermediate portion of the branch pipe 82.

The pipe 81 and the branch pipe 82 are each composed of a tube formed of a flexible material, for example, polyvinyl chloride, polyolefin such as polyethylene, polypropylene, and ethylene-vinyl acetate copolymer (EVA), polyamide, polyester, silicone rubber, and polyurethane.

The housing 5 is provided with a ventilation passage 59 for communication between the chip installing part 50 and the exterior, and one end of the pipe 81 is connected to the ventilation passage 59. Besides, the pump 80 is disposed at the other end portion of the pipe 81.

The solenoid valve 83 is for opening and closing the conduit of the branch pipe 82.

A battery 9 disposed inside the body 2 is electrically connected to the pump 80, the solenoid valve 83, the control means 11, the display part 12 and the like, to supply electric power necessary for the operations of these components.

When the pump 80 is operated in the condition where the distal opening 162 is closed by the blood sampled portion 900 in the chip installed condition and the solenoid valve 83 is in its closed state, air inside the chip installing part 50 is sucked and exhausted by the pump 80 through the ventilation passage 59 and the pipe 81, whereby the inside of the chip installing part 50 is brought into a reduced pressure state.

With the solenoid valve 83 opened (put into its open state) under this condition, air (the atmospheric air) is introduced from the exterior into the chip installing part 50 in the reduced pressure state through the branch pipe 82, the pipe 81 and the ventilation passage 59, whereby the reduced pressure state is canceled or moderated.

Incidentally, the pump 80 may be any one that can bring the inside of the chip installing part 50 into a reduced pressure state to such an extent (for example, about 100-400 mmHg) as to allow blood to be sucked out from the pierced portion of the blood sampled portion 900.

Piercing means (not shown) for moving the piercing needle 14 in the distal direction so that the needle tip of the needle body 141 passes through the distal opening 162 to pierce the blood sampled portion 900 is provided on the proximal side of the chip 13. The piercing means is composed, for example, of a spring and a plunger biased by the spring.

As has been described above, the present invention is characterized in that the contact part 3 is provided. This point (characteristic) will be described in detail below.

The contact part 3 is so provided as to surround an opening 212 of the body 2; as a result, the contact part 3 is provided at a roughly central portion thereof with an opening 30 which has a roughly circular shape in cross section.

Of the contact part 3, the surface fronting on the opening 30 constitutes a pressed surface (inner surface) 31 against which the blood sampled portion 900 is pressed. The pressed surface 31 is tilted at a substantially constant angle (θ in Fig. 3) so as to become near to the center axis of the opening 212 of the body 2 as the opening 212 is approached. In other words, the pressed surface 31 is in the shape of a side surface of a truncated cone (the shape of a portion exclusive of a top portion of a conical surface).

This configuration ensures that when a side portion of a hand (blood sampled portion 900) is pressed against the contact part 3 at the time of piercing the blood sampled portion 900, the surroundings of the blood sampled portion 900 are deformed along the pressed surface 31, whereby the shape of the surroundings is determined (see Fig. 4). A part of the blood sampled portion 900 projects into the inside of the body 2 through the opening 212.

In this case, irrespectively of the magnitude of the force with which the side portion of the hand is pressed against the contact part 3, the shape of the surroundings of the blood sampled portion 900 (particularly, the shape of the part projecting into the inside of the body 2) is determined substantially constantly by the contact part 3. Therefore, the depth of piercing of the blood sampled portion 900 by the piercing needle 14 can be made substantially constant. This makes it possible to accurately secure the blood amount required in measuring the blood sugar level.

Such an effect is sufficiently displayed by lightly putting the side portion of the hand onto the contact part 3. Particularly, the component measuring apparatus 1 in this embodiment is of the vertical installation type, so that the effect is sufficiently displayed by lightly placing the hand on the contact part 3. Therefore, at the time of measuring the blood sugar level, it is not necessary to regulate the force with which the side portion of the hand is pressed against the contact part 3, whereby enhancement of convenience for the patient can be achieved.

In addition, since the shape of the vicinity of the blood sampled portion 900 is determined, an effect of collecting blood to the blood sampled portion 900 is also displayed. This makes it possible to secure the blood required for the measurement of blood sugar level, more assuredly. In other words, the contact part 3 can be said to be a member having a blood-gathering function (blood gathering member).

Here, as shown in Fig. 3, let the minimum diameter of the opening 30 (diameter of the proximal end) be φA [mm] and let the maximum diameter of the opening 30 (diameter of the distal end) be φB [mm], then it is preferable for φB/φA to be in the range of 1.1 to 2.0, more preferably 1.2 to 1.6. Specifically, φA is preferably about 5 to 20 mm, more preferably about 8 to 15 mm.

In addition, the angle θ between the pressed surface 31 and the center axis O of the opening 30 (opening 212) is preferably about 45 to 85°, more preferably about 65 to 75°.

Such a contact part 3 has a hardness (according to JIS K 6253) of preferably not less than 30. By setting the contact part 3 to have a comparatively high hardness, deformation thereof when a side portion of a hand is pressed against it can be prevented, and, as a result, the function of the contact part 3 to determine the shape of the vicinity of the blood sampled portion 900 is displayed more preferably.

Incidentally, the pressed surface (inner surface) 31 is not limited to the one configured to be tilted at a roughly constant tilting angle over the entire length of the opening 30 as shown in the figures; for example, the pressed surface (inner surface) 31 may have a portion substantially parallel to the center axis O of the opening 212, or may be such that the tilting angle thereof varies stepwise.

Besides, in this embodiment, as shown in Figs. 3 and 4, the contact part 163 of the chip 13 is configured not to project from the opening 212 of the body 2 in the chip installed condition. This configuration ensures that in the condition where a side portion of a hand (blood sampled portion 900) is pressed against the contact part 3 (pressed surface 31), that portion of the blood sampled portion 900 which projects into the body 2 abuts on the distal end of the contact portion 163 of the chip 13 (the edge portion of the distal opening 162). By this, the blood sampled portion 900 is positioned relative to the piercing needle 14, at two positions, i.e., the contact part 3 and the contact part 163. Therefore, dispersion in the depth of piercing of the blood sampled portion 900 by the piercing needle 14 can be prevented more securely.

In addition, the opening area of the distal opening 162 of the chip 13 (chip body 130) is set to be smaller than the opening area of the opening 212 of the body 2. Therefore, in the condition where a side portion of a hand is pressed against the contact part 3, the distal opening 162 of the chip 13 is securely sealed with the blood sampled portion 900. This makes it possible to securely perform the pressure reduction by the pressure reducing means 8.

Particularly, in this embodiment, in the condition before the body fluid sampled portion is pierced by the piercing needle 14, the edge portion of the distal opening 162 of the chip 13 is set to be roughly located on an extension plane of the pressed surface (inner surface) 31 of the contact part 3 (on roughly the same plane as the pressed surface 31). This ensures that the effect of the blood sampled portion 900 to seal the distal opening 162 of the chip 13 can be displayed more securely.

Now, the method of using the component measuring apparatus 1, i.e., the operations of the individual portions and the control actions of the control means in the case of performing piercing, blood sampling (these constitute the body fluid sampling method of the present invention), development and blood sugar level measurement by use of the component measuring apparatus 1 will be described below referring to Figs. 1 to 6.
[1] First, the cover 20 is opened, and the chip 13 is inserted through the opening 212 of the body 2 into the chip installing part 50 of the housing 5. Further, the chip 13 is pushed in the proximal direction, whereon the flange 164 of the chip 13 is brought into close contact with the seal ring 55 and abuts on the distal end of the housing 5, whereby the chip 13 is positioned relative to the housing 5.
[2] Next, a power supply switch (not shown) is turned ON. This starts the individual portions of the component measuring apparatus 1, resulting in a condition allowing measurement. Incidentally, the solenoid valve 83 is in a closed state.
[3] Subsequently, a side portion of a hand (blood sampled portion 900) is pressed into close contact with the pressed portion 31 of the contact part 3. As a result, the shape of the vicinity of the blood sampled portion 900 is determined by the contact part 3, and that portion of the blood sampled portion 900 which projects into the inside of the body 2 is pressed against the contact part 163 of the chip 13. Incidentally, since the component measuring apparatus 1 in this embodiment is of the vertical installation type, the simple operation of placing the hand on the contact part 3 ensures that the distal opening 162 of the chip 13 is accurately closed with the blood sampled portion 900.
[4] Next, the operating button 216 is depressed, to cause piercing of the surface (skin) of the blood sampled portion 900 (step S101 in Fig. 6).
   With the operating button 216 depressed, the above-mentioned piercing means is operated to move the piercing needle 14 in the distal direction, and the needle body 141 passes through the hole 151 to project from the distal opening 162, thereby piercing the surface of the side portion of the hand (body fluid sampled portion). This causes bleeding from the portion pierced by the needle body 141.
   In this instance, the blood sampled portion 900 is positioned relative to the piercing needle 14 by the contact part 3 and the contact part 163 of the chip 13, so that piercing of the blood sampled portion 900 is conducted with an accurate piercing depth.
   In addition, with the operating button 216 depressed, an operating switch (not shown) for the pump 80 is also turned ON substantially simultaneously.
[5] After the blood sampled portion 900 is pierced by the needle body 141, the piercing needle 14 is returned in the proximal direction, and the needle tip of the needle body 141 is stored into the chip 13.
[6] When the operating switch for the pump 80 is turned ON, the control means 11 starts the operation of the pump 80 (step S102 in Fig. 6).
   Specifically, the pump 80 operates substantially simultaneously with the operation of [4] above, and suction and evacuation of air inside the chip installing part 50 of the housing 5 is started. As a result, the pressure inside the chip installing part 50 (inclusive of the inside of the chip 13) is lowered, resulting in a reduced pressure state.
   In this instance, that portion of the blood sampled portion 900 which is pierced by the needle body 141 is also in a reduced pressure state. Therefore, blood is sucked out from the pierced portion, and bleeding is accelerated, so that a required blood amount can be secured in a short time.
   Incidentally, the lowest pressure generated by such a pump 80 is preferably about 100 to 400 mmHg, for example.
   As has been described above, in the component measuring apparatus 1, with the operating button 216 depressed once, the piercing action and the pressure reducing action are performed substantially simultaneously, which means an extremely favorable operability.
[7] The blood protuberant in the shape of a particle on the pierced portion of the side portion of the hand (blood sampled portion 900) in the operation of [6] above is sucked into the chip 13 to make contact with the blood introduction guide 166 formed inside the chip 13, is guided through the blood passage 19 to the test paper 18, is supplied to a central area of the test paper 18, and is developed radially.
   Attendant on the supply of the blood onto the test paper 18 and development thereon, the glucose (the component to be measured) in the blood and the reagent supported on the test paper 18 react with each other, and the test paper 18 shows coloration according to the quantity of glucose.
   On the other hand, the control means 11 executes step S102 shown in Fig. 6, drives the measuring means 7, monitors the coloration of the test paper 18 through the measuring means 7, and judges whether or not the blood has been sampled (step S103 in Fig. 6).
   In step S103, where the voltage level of a signal inputted from the light receiving device 72 of the measuring means 7 exceeds a preset threshold, it is judged that the blood has been sampled, and where the voltage level of the signal is not more than the threshold, it is judged that the blood has not yet been sampled.
   Incidentally, the threshold is set at a value sufficiently higher than the voltage level of the signal before the coloration of the test paper 18, and sufficiently lower than the voltage level of the signal upon the coloration.
   When it is judged in step S103 that the blood has not yet been sampled, it is judged whether or not the time is up (step S104 in Fig. 6).
   When it is judged in step S104 that the time is not yet up, the process returns to step S103, then step S103 and the like are again executed, and when it is judged that the time is up, an error processing is carried out (step S105 in Fig. 6).
   In step S105, the pump 80 is stopped, the solenoid 83 is opened, to cancel the reduced pressure state, and an error indication is displayed on the display part 12.
   With the error indication, the operator (user) can grasp the presence of an error (that some trouble has occurred).
   Incidentally, the action when the solenoid valve 83 is opened will be described in detail later.
   Besides, when it is judged in step S103 that the blood has been sampled, the pump 80 is stopped (step S106 in Fig. 6).
   Next, the solenoid valve 83 is opened, to cancel the reduced pressure state (step S107 in Fig. 6).
   With the solenoid valve 83 opened, the outside air (atmospheric air) flows into the chip installing part 50 (inclusive of the inside of the chip 13) and to the pierced portion of the blood sampled portion 900 through the branch pipe 82, the pipe 81 and the ventilation passage 59, thereby causing a return to the atmospheric pressure.
   When the feeling of suction in the vicinity of the pierced portion of the side portion of the hand is eliminated and the return to the atmospheric pressure is confirmed, the hand is put off the contact part 3.
[8] After executing step S107 in Fig. 6, the control means 11 measures the degree of coloration of the test paper 18 by the measuring means 7, performs an arithmetic processing based on the data obtained, and carries out corrections such as a temperature correction calculation, and a hematocrit value correction calculation, thereby quantitatively determining the blood sugar level (step S108 in Fig. 6).

In this case, since the reduced pressure state of the chip installing part 50 (inclusive of the inside of the chip 13), i.e., the reduced pressure state in the storing space for the test paper 18 has been canceled, the atmospheric air components (for example, oxygen, carbon dioxide, and water vapor) necessary for the reaction between the glucose (component to be measured) in the blood and the reagent supported on the test paper 18 are sufficiently supplied, whereby the blood sugar level can be measured accurately.

Next, the blood sugar level thus calculated is displayed on the display part 12 (step S109 in Fig. 6). This makes it possible to know the blood sugar level.

Incidentally, after the reduced pressure state is canceled, the solenoid valve 83 is again closed, as a preparation for the next round of measurement.

As has been described above, according to the component measuring apparatus 1, the blood sampled portion 900 is positioned relative to the piercing needle 14 by the contact part 3 and the contact part 163 of the chip 13, whereby dispersion in the depth of piercing is restrained. This makes it possible to securely sample the blood in an amount necessary and sufficient for measurement, and, as a result, to measure the blood sugar level (the quantity of a predetermined component in blood) accurately and securely.

In addition, since the chip 13 is provided with the test paper 18, piercing, blood sampling and development onto the test paper 18, and measurement (quantitative determination of the component) can be carried out continuously, and the blood sugar level measurement (component measurement) can be performed easily and speedily.

Besides, where the measuring apparatus is of the vertical installation type, the apparatus can be used easily; for example, the measurement of blood sugar level can be started by only placing the blood sampled portion (body fluid sampled portion) 900 on the contact part 3. Therefore, the apparatus is advantageous in the case of periodical use and in the case of repeated use.

In addition, such accidents as erroneously piercing the living body surface again after piercing once are prevented from occurring, and safety is high. Moreover, since the piercing needle 14 cannot be seen directly, the sensation of fear at the time of piercing is alleviated.

From the foregoing, the component measuring apparatus 1 is suited to use in measuring the blood sugar value or the like of a patient by the patient himself.

Besides, the component measuring apparatus 1 is simple in configuration, small in size and weight, inexpensive, and suited to mass production.

While the body fluid sampling implement and the body fluid sampling method according to the present invention have been described above based on the embodiments shown in the drawings, the present invention is not limited to or by the embodiments; for example, the configuration of each portion can be replaced by an arbitrary configuration capable of displaying an equivalent function.

For example, while the measuring means is for measuring the quantity of a predetermined component, it may be for measuring a property of a predetermined component, or may be for measuring both the quantity and a property of a predetermined component.

In addition, the measuring means is not limited to the one for optically measuring the intensity of coloration of a test paper showing a coloration due to a reaction between a component in blood and a reagent (colorimetry), converting the intensity of coloration into a measured value, and displaying the measured value; for example, the measuring means may be one for electrically measuring a variation in potential generated according to the quantity of a component in a specimen, converting the potential variation into a measured value, and displaying the measured value.

Besides, while the pressure reducing means is so configured as to cancel the reduced pressure state prior to the measurement, it may be so configured as to moderate the reduced pressure state prior to the measurement.

In addition, the pressure reducing means is so configured as to start pressure reduction prior to piercing.

Besides, the pressure reducing means may be started manually or be started automatically. In the latter case, a configuration may be adopted in which, for example, a sensor or the like designed to magnetically sense the movement of a piercing needle in the distal direction at the time of piercing a fingertip is disposed in a lateral vicinity of a distal end portion of the housing 5, and the pressure reducing means is operated based on information from the sensor.

In addition, the component measuring apparatus 1 may be provided with a chip retracting mechanism for moving the chip 13 in the proximal direction.

Incidentally, the case in which the body fluid sampling implement of the present invention is applied to a component measuring apparatus having both the function to sample blood and the function to measure the quantity of a predetermined component in the blood has been described in the above embodiments, it is natural that, in the present invention, the function to measure the quantity of a predetermined component in blood may be omitted.

Besides, while blood has been described as a representative of the body fluid to be sampled in the above embodiments, the body fluid to be sampled is not limited to blood, and may be, for example, sweat, lymph, interstitial fluid, and cerebrospinal fluid.

In addition, while glucose (blood sugar level) has been described as a representative of the component to be measured in the above embodiments, the component to be measured in the present invention is not limited to glucose (blood sugar level), and may be, for example, an organic compound such as protein, cholesterol, uric acid, creatinine, and alcohol, or an inorganic ion such as sodium, potassium, calcium, chloride, phosphate, carbonate, and pH.

### Industrial Applicability

According to the present invention, the shape of the vicinity of the body fluid sampled portion is determined by the contact part, so that dispersion of the depth of piercing is restrained, and it is possible to sample a body fluid more accurately. In addition, the effect can be further enhanced by configuring the body fluid sampling implement in the condition where the chip having the piercing needle is mounted, and appropriately setting the positional relationship, size relationship or the like, between the contact part and the distal end of the chip. Besides, the body fluid sampling implement according to the present invention is simple in configuration, small in size and weight, inexpensive, and suited to mass production. Therefore, the body fluid sampling implement according to the present invention has industrial applicability.

## Claims

1. A body fluid sampling implement used by installing a piercing needle therein and allowing a body fluid to be discharged by piercing a body fluid sampled portion with said piercing needle, comprising:
a body storing said piercing needle and having an opening allowing said piercing needle to pass therethrough; and
a contact part fixedly installed on said body so as to surround the outer periphery of said opening and pressed by said body fluid sampled portion when said body fluid sampled portion is pierced by said piercing needle,
wherein the inner surface of said contact part has a portion tilted so as to near the center axis of said opening as said opening is approached.

2. The body fluid sampling implement as set forth in claim 1, wherein said inner surface of said contact part is in the shape of a truncated cone in side view.

3. The body fluid sampling implement as set forth in claim 1 or 2, wherein:
said piercing needle is mounted to said body fluid sampling implement as a chip stored in a chip body having an opening allowing said piercing needle to pass therethrough; and
said body fluid sampled portion abuts on an edge portion of said opening of said chip body when said body fluid sampled portion is pressed against said contact part in the condition where said chip is mounted to said body fluid sampling implement.

4. The body fluid sampling means as set forth in claim 3, wherein an opening area of said opening of said chip body is smaller than an opening area of said opening body said body.

5. The body fluid sampling implement as set forth in claim 3, wherein said edge portion of said opening of said chip body is located substantially on an extension plane of said tilted portion of said inner surface of said contact part, in the condition before said body fluid sampled portion is pierced with said piercing needle.

6. The body fluid sampling implement as set forth in claim 1, used in the condition where said opening of said body is directed vertically upward.

7. A body fluid sampling method for sampling a body fluid by piercing a body fluid sampled portion with a piercing needle by use of a body fluid sampling implement comprising:
a body storing said piercing needle and having an opening allowing said piercing needle to pass therethrough; and
a contact part fixedly installed on said body so as to surround the outer periphery of said opening and pressed by said body fluid sampled portion when said body fluid sampled portion is pierced by said piercing needle,
the inner surface of said contact part having a portion tilted so as to near the center axis of said opening as said opening is approached,
wherein said body fluid is discharged by piercing said body fluid sampled portion with said piercing needle in the condition where said body fluid sampled portion is pressed against said contact part.

8. A body fluid sampling method carried out by use of a body fluid sampling implement including:
a body storing said piercing needle and having an opening allowing said piercing needle to pass therethrough; and
a contact part fixedly installed on said body so as to surround the outer periphery of said opening and pressed by said body fluid sampled portion when said body fluid sampled portion is pierced by said piercing needle,
the inner surface of said contact part having a portion tilted so as to near the center axis of said opening as said opening is approached,
said method comprising the steps of:
pressing said body fluid sampled portion into close contact with said inner surface of said contact part,
operating said piercing needle so as to pierce with said piercing needle the portion of said body fluid sampled portion which is projected into said body through said opening; and
sampling the body fluid discharged from the pierced portion of said body fluid sampled portion.

9. The body fluid sampling method as set forth in claim 8, wherein said inner surface of said contact part is in the shape of a truncated cone in side view.

10. The body fluid sampling method as set forth in claim 7 or 8, wherein:
said piercing needle is mounted to said body fluid sampling implement as a chip stored in a chip body having an opening allowing said piercing needle to pass therethrough; and
said body fluid sampled portion abuts on an edge portion of said opening of said chip body when said body fluid sampled portion is pressed against said contact part in the condition where said chip is mounted to said body fluid sampling implement.

11. The body fluid sampling method as set forth in claim 10, wherein the opening area of said opening of said chip body is smaller than the opening area of said opening of said body.

12. The body fluid sampling method as set forth in claim 10, wherein said edge portion of said opening of said chip body is located substantially on an extension plane of said tilted portion of said inner surface of said contact part, in the condition before said body fluid sampled portion is pierced with said piercing needle.

13. The body fluid sampling method as set forth in claim 7 or 8, wherein said body fluid sampling implement is used in the condition where said opening of said body is directed vertically upward.
